# FASCICULE DE BREVET EUROPEEN

(11) **EP 1 559 393 B1**
(45) Date de publication et mention de la délivrance du brevet: **06.05.2009**
(21) Numéro de dépôt: 05290186.5
(22) Date de dépôt: 27.01.2005
(51) Int. Cl.: A61K 8/92, A61K 8/25, A61K 8/88, A61K 8/81, A61Q 1/00, A61Q 1/12

(54) **Composition cosmétique de type poudre compacte**
Kosmetische Kompaktpuderzusammenstzung
Pressed powder cosmetic composition

(30) Priorité: 27.01.2004 FR 0450150
(43) Date de publication de la demande: 03.08.2005
(73) Titulaire: L'ORÉAL, 75008 Paris (FR)
(72) Inventeur: Martin, Guenaelle, 78360 Montesson (FR); Themens, Agnès, 92340 Bourg la Reine (FR); Collineau, Maitena, 75005 Paris (FR)
(74) Mandataire: Le Coupanec, Pascale A.M.P.

(56) Documents cités:
- EP-A- 0 524 892
- EP-A- 0 605 284
- EP-A- 0 717 979
- EP-A- 1 159 950
- EP-A- 1 277 458
- WO-A-02/03935
- WO-A-96/17583
- FR-A- 2 798 287
- FR-A- 2 812 194
- FR-A- 2 854 324
- DATABASE CAPLUS [Online] XP002294996 extrait de STN Database accession no. 1999:344564 & JP 11 147809 A (KAO CORP.) 2 juin 1999 (1999-06-02)

## Description

La présente invention concerne une composition cosmétique pour le maquillage et/ou le soin de la peau, notamment du visage, se présentant sous forme de poudre compacte.

Plus particulièrement, les compositions considérées selon l'invention peuvent constituer un produit de maquillage, par exemple du visage, ayant notamment des propriétés de soin et/ou de traitement non thérapeutique et en particulier destiné à réduire la brillance des peaux dites grasses et/ou améliorer la tenue du maquillage à long terme, c'est-à-dire prévenir la dégradation visuelle au cours de la journée. Ce type de composition est encore qualifiée de composition cosmétique matifiante.

Dans le domaine des compositions cosmétiques poudreuses sous forme de poudres compactes, il est généralement utilisé, d'une part une phase particulaire comportant notamment des pigments et/ou des charges, et d'autre part une phase grasse comprenant des corps gras, à titre de liant. Ce liant est notamment destiné à procurer une certaine cohésion à la phase particulaire desdites poudres, de manière à diminuer les risques de fragmentation de celles-ci sous l'impact de chocs et à en assurer un prélèvement aisé.

A titre illustratif des liants utilisés dans des poudres compactes on peut notamment citer ceux décrits dans les documents suivants.

La demande PCT WO 93/17660 décrit des compositions cosmétiques sous forme de poudre contenant, à titre de liant, un liant gras siliconé constitué par au moins une huile de silicone, au moins une cire de silicone, au moins une résine de silicone, et éventuellement au moins une gomme de silicone et au moins une phényldiméthicone.

Le document EP 0 717 979 décrit un procédé de préparation d'une composition comprenant une phase grasse majoritairement liquide, voire essentiellement liquide, en particulier à base d'huile de parleam, et une phase pulvérulente, selon lequel la phase pulvérulente est dispersée dans une émulsion huile-dans-eau, la dispersion ainsi obtenue est coulée dans un moule, puis séchée par lyophilisation.

Le document EP 0 792 633 concerne l'utilisation d'esters liquides ayant une mouillabilité allant de 30 secondes à dix minutes, comme par exemple le triisostéarate de glycéryle, comme liants de compositions sous forme de poudres, notamment compactées.

Le brevet EP 0 923 927 décrit des compositions pulvérulentes à application topique comprenant au moins un composé pulvérulent dont les particules sont liées entre elles par un liant comprenant une phase grasse liquide et au moins 2 % en poids de polymère dispersible dans ladite phase grasse liquide.

D'une manière générale, les liants utilisés se présentent donc sous une forme liquide. Or cette formulation peut s'avérer, dans le cas des compositions cosmétiques poudreuses qualifiées matifiantes, préjudiciable à la manifestation d'un effet matifiant d'une manière prolongée dans le temps.

Comme précisé précédemment, un produit matifiant est un produit qui empêche la peau de briller et qui unifie le teint. Pour se faire, il comprend généralement au moins un composé pulvérulent d'origine naturelle ou synthétique capable notamment d'absorber le sébum. Ces composés sont encore dénommés « pompes à sébum ».

En fait, le liant liquide peut être absorbé au moins en partie par les composés de type pompe à sébum et à ce titre affecter la capacité de ceux-ci à absorber et/ou adsorber le sébum auquel ils sont plus particulièrement dédiés.

Cette absorption « parasite » est d'une part préjudiciable à l'obtention d'un effet matifiant prolongé dans le temps et d'autre part peut induire une inhomogénéité de couleur au niveau du maquillage.

Il existe donc un besoin pour une composition cosmétique sous forme de poudre compacte présentant à la fois des propriétés de cohésion et de délitage satisfaisantes et permettant d'obtenir un maquillage présentant une bonne capacité matifiante et une bonne tenue de matité dans le temps.

Les inventeurs ont constaté qu'il était possible de remédier à l'inconvénient précité, sans pour autant porter préjudice aux propriétés de cohésion et de délitage des poudres compactes sous réserve du choix d'un système liant spécifique.

En l'occurrence, l'invention concerne selon un de ses aspects, une composition cosmétique sous forme de poudre compacte pour le maquillage et/ou le soin de la peau, notamment du visage, comprenant au moins un composé absorbant et/ou adsorbant le sébum et au moins une phase grasse, ladite phase grasse comprenant, à raison de plus de 40 % en poids de son poids total, une phase grasse solide, et ladite composition étant caractérisée en ce que la phase grasse totale est présente en une teneur inférieure ou égale à 35% en poids par rapport au poids total de la composition.

Selon une variante particulière, la composition selon l'invention ne peut être une composition comprenant 10 % en poids de cire de Carnauba, 6 % en poids de stéarate de magnésium et 5 % en poids de silicate de magnésium et d'aluminium amorphe, par rapport au poids total de la composition.

Selon un autre de ses aspects, l'invention a encore pour objet l'utilisation d'au moins une phase grasse solide et d'au moins un composé absorbant et/ou adsorbant le sébum dans une composition cosmétique de type poudre compacte pour obtenir un maquillage homogène en couleur et/ou en matité.

Selon un autre de ses aspects, l'invention a également pour objet un procédé de maquillage et/ou de soin de la peau comprenant au moins une étape d'application sur la peau d'au moins une composition selon l'invention.

Selon un autre de ses aspects, l'invention a aussi pour objet l'utilisation d'une composition selon l'invention pour obtenir un maquillage de la peau homogène en couleur et/ou en matité.

Comme précisé précédemment, les compositions selon l'invention se présentent sous la forme d'une poudre compacte. Cet aspect compacté est obtenu en soumettant à une compression le mélange phase pulvérulente et liant gras associé.

Plus particulièrement, les compositions sous forme de poudre compacte conformes à l'invention peuvent être préparées en mélangeant l'ensemble des composants de la phase pulvérulente (charges et pigments) puis en ajoutant à ce mélange les composés constituant la phase grasse sous agitation. Le mélange est ensuite broyé, tamisé, puis versé dans une coupelle et compacté. Ce compactage est en général réalisé à l'aide d'une presse notamment en appliquant une pression de 50 à 250 bars pour obtenir la poudre compacte attendue.

Par conséquent, les compositions selon l'invention sont différentes des compositions pulvérulentes qualifiées de coulées, qui sont préparées par simple mélange d'une phase grasse à l'état fondu avec une phase pulvérulente.

Le fait que les compositions se présentent sous une forme compacte confère à celles-ci, comparativement à des compositions de type coulé, une meilleure aptitude à un délitage sous forme de particules solides « libres ».

Ainsi, on peut, à l'issu d'un décompactage d'une composition compacte selon l'invention, récupérer sa phase pulvérulente, ce qui n'est pas possible pour une composition coulée.

Ce décompactage peut notamment être réalisé selon le protocole suivant : on casse le produit compacté à l'aide d'une spatule au dessus d'un tamis (250µ) puis on tamise les paquets de poudre obtenus. On récupère ainsi la phase pulvérulente de la poudre compacte sous forme de particules solides « libres ».

### Composé absorbant le sébum :

Les compositions selon l'invention comprennent au moins un composé absorbant le sébum, encore appelé « pompe à sébum ».

On entend par « composé absorbant le sébum », un composé apte à absorber et/ou adsorber le sébum. Généralement ce type de composé se présente sous la forme d'une poudre ayant une prise de sébum.

De manière avantageuse, la prise de sébum de ces composés est supérieure ou égale à 1 ml/g, et peut notamment varier de 1 ml/g à 20 ml/g, en particulier de 1 ml/g à 15 ml/g. Elle peut notamment être supérieure ou égale à 1,5 ml/g, et en particulier varier de 1,5 ml/g à 20 ml/g, voire varier de 1,5 ml/g à 15 ml/g.

Selon une variante particulière, la prise de sébum dudit composé peut être supérieure ou égale à 2 ml/g, et peut notamment varier de 2 ml/g à 20 ml/g, et en particulier de 2 ml/g à 15 ml/g.

La prise de sébum correspond à la quantité de sébum absorbée et/ou adsorbée sur la surface disponible des particules. Elle est mesurée selon la méthode de Wet Point décrite ci après dans les exemples.

Selon un mode de réalisation particulier, les particules de composé(s) absorbant et/ou adsorbant le sébum peuvent présenter une surface spécifique BET supérieure ou égale à 300 m²/g, notamment supérieure ou égale à 500 m²/g, et en particulier supérieure ou égale à 600 m²/g, et notamment inférieure ou égale à 1500 m²/g.

Les particules de composé(s) absorbant et/ou adsorbant le sébum peuvent être d'origine minérale ou organique. Elles peuvent notamment être choisies parmi : la silice, les poudres de polyamide (nylon®), les poudres de polymères acryliques, notamment de polyméthacrylate de méthyle, de polyméthacrylate de méthyle/diméthacrylate d'éthylène glycol, de polyméthacrylate d'allyle/diméthacrylate d'éthylène glycol de copolymère diméthacrylate d'éthylène glycol/méthacrylate de lauryle et les poudres de polyéthylène, notamment de polyéthylène/acide acrylique.

Les particules de ce composé peuvent, le cas échéant, être traitées en surface par au moins un agent de traitement hydrophobe.

Cet agent de traitement hydrophobe peut notamment être choisi parmi :
- les silicones, comme les méthicones, les diméthicones ;
- les acides gras, comme l'acide stéarique ;
- les savons métalliques, comme le dimyristate d'aluminium, le sel d'aluminium du glutamate de suif hydrogéné ;
- les perfluoroalkyl phosphates, les perfluoroalkyl silanes, les perfluoroalkyl silazanes, les polyoxydes d'hexafluoropropylène, les polyorganosiloxanes comprenant des groupes perfluoroalkyl perfluoropolyéthers ;
- les acides aminés, les acides aminés N-acylés ou leurs sels ;
- la lécithine, le triisostéaryle titanate d'isopropyle ; et
- leurs mélanges.

Le terme « alkyle » mentionné dans les composés cités précédemment peut désigner notamment un groupe alkyle, linéaire, ramifié ou cyclique, comprenant de 1 à 30 atomes de carbone, notamment de 5 à 16 atomes de carbone.

Les acides aminés N-acylés peuvent comprendre un groupe acyle comprenant de 8 à 22 atomes de carbone, comme par exemple un groupe 2-éthyl hexanoyle, caproyle, lauroyle, myristoyle, palmitoyle, stéaroyle et cocoyle.

Les sels de ces composés peuvent être les sels d'aluminium, de magnésium, de calcium, de zirconium, de zinc, de sodium ou de potassium.

L'acide aminé peut être par exemple la lysine, l'acide glutamique ou l'alanine.

A titre représentatif et non limitatif des composés absorbant et/ou adsorbant le sébum selon l'invention, on peut tout particulièrement citer :
- les poudres de silice, comme par exemple les microsphères de silice poreuses vendues sous la dénomination "SILICA BEADS SB-700" commercialisées par la société MYOSHI, les "SUNSPHERE® H51 ", "SUNSPHERE® H33" , "SUNSPHERE® H53" commercialisées par la société ASAHI GLASS ; et les microsphères de silice amorphe enrobées de polydiméthylsiloxane vendues sous la dénomination "SA SUNSPHERE® H-33" et "SA SUNSPHERE® H-53" par la société ASAHI GLASS,
- les poudres de polyamides (nylon®), comme par exemple l'"ORGASOL® 4000" ou l'"ORGASOL® 2002 EXTRA D NAT COS" commercialisés par la société ATOCHEM,
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING, et
- les poudres de polyéthylène, notamment de polyéthylène/acide acrylique vendues sous le nom commercial Flobeads® par la société SUMITOMO.

Selon une variante particulière, le composé absorbant et/ou adsorbant le sébum est d'origine minérale.

Selon un mode de réalisation tout particulièrement avantageux, les particules de composé absorbant et/ou adsorbant le sébum sont choisies parmi les particules de silice, les particules de polyéthylène, les poudres de polyamide et leurs mélanges. En particulier, les particules de composé absorbant et/ou adsorbant le sébum sont des particules de silice. Cette silice peut présenter notamment les caractéristiques décrites précédemment, et en particulier posséder une prise de sébum supérieure ou égale à 2 ml/g, notamment variant de 2 ml/g à 20 ml/g. De telles particules de silice sont notamment vendues sous les dénominations "SUNSPHERE® H 33" et "SUNSPHERE® H53" par la société ASAHI GLASS.

Les compositions selon l'invention peuvent comprendre le composé absorbant et/ou adsorbant le sébum en une teneur variant de 2 à 18 % en poids, notamment de 4 à 18 % en poids, en particulier de 5 à 15 % en poids, par rapport au poids total de la composition.

### Phase grasse :

Comme précisé précédemment la phase grasse est encore communément appelée liant et sert notamment de milieu dispersant pour la phase particulaire. Cette phase grasse peut être présente dans la composition selon l'invention en une teneur allant de 12 à 35 % en poids, et notamment de 15 à 30 % en poids par rapport au poids total de la composition.

### Phase grasse solide :

La composition selon l'invention comporte au moins une phase grasse comprenant à raison de plus de 40 % en poids de son poids une phase grasse solide, dite encore « liant solide ».

Par « liant solide », on entend au sens de la présente invention une phase grasse dont le point de fusion peut être supérieur ou égal à 30 °C, notamment peut varier de 30 à 250 °C et en particulier de 30 à 230 °C.

Cette phase grasse solide peut comprendre au moins un composé choisi parmi les cires, les savons métalliques et leurs mélanges.

Par "cire", au sens de la présente invention, on entend un composé gras lipophile, solide à température ambiante (25 °C) et pression atmosphérique (760 mm Hg, soit 10⁵ Pa), à changement d'état solide/liquide réversible, ayant en particulier une température de fusion supérieure ou égale à 30 °C, notamment supérieure ou égale à 55 °C, et pouvant aller jusqu'à 250 °C, notamment jusqu'à 230 °C, et en particulier jusqu'à 120 °C.

En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.

Les cires, au sens de l'invention, peuvent être celles utilisées généralement dans les domaines cosmétiques ou dermatologiques. Elles peuvent notamment être hydrocarbonées, siliconées et/ou fluorées, comportant éventuellement des fonctions ester ou hydroxyle. Elles peuvent être également d'origine naturelle ou synthétique.

A titre illustratif et non limitatif de ces cires, on peut notamment citer :
- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges.

Selon une variante particulière de l'invention, la phase grasse solide comprend au moins un composé choisi parmi la cire de carnauba, la cire de paraffine et leurs mélanges.

Selon une autre variante particulière, la phase grasse solide peut comprendre au moins une cire présente totalement ou partiellement sous forme de poudre, notamment micronisée, pour faciliter sa mise en oeuvre dans la préparation de la composition cosmétique.

Parmi les cires utilisables sous forme de poudre, on peut notamment citer les microbilles de cire de Carnauba vendues sous la dénomination MICROCARE 350® par la société Micro Powders et les microbilles de cire de paraffine vendues sous la dénomination MICROEASE 114S® par la société Micro Powders.

Le liant solide peut également être choisi parmi les savons métalliques.

Parmi ceux-ci, on peut notamment citer les savons métalliques d'acides gras ayant de 12 à 22 atomes de carbone, et en particulier ceux ayant de 12 à 18 atomes de carbone.

Le métal du savon métallique peut notamment être du zinc ou du magnésium.

L'acide gras peut notamment être choisi parmi l'acide laurique, l'acide myristique, l'acide stéarique, l'acide palmitique, et leurs mélanges.

Comme savon métallique, on peut utiliser le laurate de zinc, le stéarate de magnésium, le myristate de magnésium, le stéarate de zinc, et leurs mélanges.

Selon une variante particulière de l'invention, la phase grasse solide peut comprendre au moins un savon métallique présent totalement ou partiellement sous forme de poudre.

La phase grasse solide peut être présente en une teneur supérieure ou égale à 45 % en poids, notamment supérieure ou égale à 50 % en poids, en particulier supérieure ou égale à 55 % en poids, plus particulièrement supérieur ou égal à 60 % en poids, encore plus particulièrement supérieur ou égal à 70 %, voire supérieur ou égal à 80 % en poids par rapport au poids total de la phase grasse.

Selon une variante particulière, ladite phase grasse solide peut constituer l'intégralité de la phase grasse.

Selon une variante particulière, le rapport pondéral phase grasse solide / pompe à sébum est au moins égal à 1, et peut notamment varier de 1 à 2,5 et en particulier de 1 à 2,3.

La composition selon l'invention peut ainsi comprendre au moins une phase grasse solide en une teneur variant de 8 à 25 % en poids, notamment de 10 à 22 % en poids, et en particulier de 11 à 20 % en poids par rapport au poids total de la composition.

Conviennent tout particulièrement à l'invention les associations pompes à sébum/phase grasse solide suivantes :
- microsphères de silice poreuses, comme par exemple les Sunsphère H-33®/cire de carnauba ou de paraffine micronisée,
- microsphères de silice poreuses, comme par exemple les Sunsphère H-33®, et poudre de polyéthylène/acide acrylique, comme par exemple les Flobeads® ZA-209®/cire de carnauba ou de paraffine micronisée et stéarate de magnésium ou stéarate de zinc,
- microsphères de silice poreuses, comme par exemple les Sunsphère H-33®, poudre de polyéthylène/acide acrylique, comme par exemple les Flobeads ZA-209®, et poudre de polyamide comme par exemple les Orgasol®/cire de carnauba ou de paraffine micronisée et stéarate de magnésium ou stéarate de zinc.

### Phase grasse liquide:

La phase grasse de la composition selon l'invention peut en outre comprendre une phase grasse liquide comportant au moins une huile. Cette huile peut être choisie parmi les huiles utilisées classiquement comme liant dans les poudres compactes.

Ces huiles peuvent être notamment choisies parmi :
- l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures, telles que les huiles de paraffine, le squalane, la vaseline, le polydécène, notamment hydrogéné, comme le « CERAFLOW E® » commercialisé par la société Shamrock ;
- les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle et le triisostéarate de glycérine ou de diglycérine ;
- les huiles de silicone telles que les polyméthylsiloxanes, les polyméthylphénylsiloxanes, les polysiloxanes modifiés par des acides gras, des alcools gras ou des polyoxyalkylènes, les silicones fluorées et les huiles perfluorées ;
- les acides gras supérieurs tels que l'acide myristique, l'acide palmitique, l'acide stéarique, l'acide béhénique, l'acide oléique, l'acide linoléique, l'acide linolénique et l'acide isostéarique;
- les alcools gras supérieurs tels que le cétanol et l'alcool oléique ;
- les polyméthylfluoroalkyl diméthylsiloxanes de formule (I) : 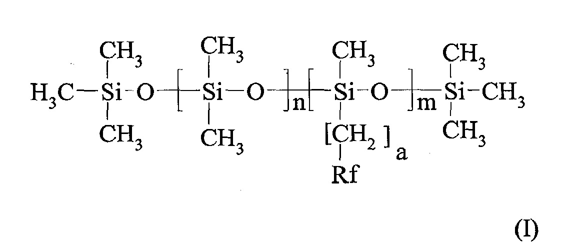
dans laquelle :
- n représente un entier variant de 5 à 90, notamment de 30 à 80 et en particulier de 50 à 80,
- m représente un entier variant de 1 à 150, notamment de 1 à 80, et en particulier de 1 à 40,
- a représente un entier variant de 0 à 5, et
- Rf désigne un radical perfluoroalkyle comprenant de 1 à 8 atomes de carbone ; et
- leurs mélanges.

Comme composés de formule (I) convenant tout particulièrement à l'invention, on peut notamment citer ceux vendus sous la dénomination X22-819®, X22-820®, X22-821®, X22-822® par la société SHIN-ETSU.

En particulier, la composition selon l'invention peut comprendre une phase grasse liquide en une teneur variant de 4 à 15 % en poids, notamment de 6 à 13 % en poids, et en particulier de 7 à 13 % en poids par rapport au poids total de la composition.

Selon une variante particulière, le rapport pondéral phase grasse liquide/pompe(s) à sébum peut être inférieur ou égal à 2, et peut notamment varier de 0,5 à 2.

Selon une variante particulière la composition selon l'invention peut être exempte de phase grasse liquide.

Bien entendu, l'homme de l'art veillera à ajuster les quantités de phase grasse solide et éventuellement liquide de la composition selon l'invention de manière telle que les propriétés attendus en termes de cohésion et d'effet matifiant dans le temps soient satisfaisantes.

### Charges :

La composition selon l'invention peut comprendre en outre au moins une charge additionnelle, c'est-à-dire distincte du ou des composé(s) pompe à sébum déjà retenu(s) dans ladite composition.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique, etc...). Parmi les charges additionnelles utilisables dans les compositions selon l'invention, on peut notamment citer le talc, le mica, la silice, le kaolin, les poudres de polyamide (Nylon®) , de poly-β-alanine et de polyéthylène, les poudres de polymères de tétrafluoroéthylène (Téflon®), la lauroyl-lysine, l'amidon, le nitrure de bore, les poudres de polymères d'acide acrylique, les microbilles de résine de silicone (Tospearls® de Toshiba, par exemple), le carbonate de calcium précipité, le carbonate et l'hydro-carbonate de magnésium, l'hydroxyapatite, les microsphères de silice creuses, et les microcapsules de céramique.

Les compositions selon l'invention peuvent comprendre au moins une charge additionnelle ou un mélange de charges additionnelles en une teneur variant de 40 à 95 % en poids, notamment de 45 à 85 % en poids, et en particulier de 45 à 70 % en poids par rapport au poids total de la composition.

### Matière colorante pulvérulente

La phase particulaire de la composition selon l'invention peut avantageusement contenir en outre au moins une matière colorante pulvérulente pouvant être choisie parmi les pigments et les nacres habituellement utilisés dans les compositions cosmétiques et/ou dermatologiques.

Les pigments peuvent être blancs ou colorés, minéraux et/ou organiques, enrobés ou non. On peut notamment citer parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut notamment citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La matière colorante pulvérulente peut généralement être présente dans la composition en une teneur variant de 0,5 à 30 % en poids, notamment de 1 à 22 % en poids, et en particulier de 3 à 18 % en poids par rapport au poids total de la composition.

### Additifs :

La composition peut également comprendre d'autres ingrédients (adjuvants) usuellement utilisés en cosmétique tels que les agents colorants hydrosolubles ou liposolubles, les conservateurs, les actifs cosmétiques, les agents hydratants, les filtres UV, les épaississants, l'eau, les tensioactifs et/ou les parfums.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention peut notamment se présenter sous la forme d'un produit de maquillage de type poudre compacte, en particulier un fard à joues, un fard à paupières, une poudre pour le visage, un fond de teint, un produit anti-cernes, un produit de maquillage du corps, ou bien encore sous la forme d'un produit de soin pour le visage, d'un produit de soin du corps.

Les exemples de compositions ci-après sont donnés à titre illustratif et sans caractère limitatif de l'invention.

### Exemples :

Les formulations suivantes sont préparées selon les procédés de préparation classiquement utilisés en cosmétique.

### Méthode de mesure de prise de sébum d'une poudre

La prise de sébum d'une poudre est mesurée selon la méthode de détermination de prise d'huile de poudre décrite dans la norme NF T 30-022. Elle correspond à la quantité de sébum adsorbée sur la surface disponible de la poudre et/ou absorbée par la poudre par mesure du Wet Point, décrite ci-dessous :

On place une quantité m (en grammes) de poudre comprise entre environ 0,5 g et 5,0 g (la quantité dépend de la densité de la poudre) sur une plaque de verre puis on ajoute goutte à goutte du sébum artificiel, maintenu à la température de 29 °C et ayant la composition suivante :
- trioléine 29,00 %
- acide oléïque 28,50 %
- oléate d'oléyle 18,50 %
- squalène 14,00 %
- cholestérol 7,00 %
- palmitate de cholestérol 3,00 %

Après addition de 4 à 5 gouttes de sébum artificiel, on incorpore le sébum artificiel dans la poudre à l'aide d'une spatule et on continue d'ajouter du sébum artificiel jusqu'à la formation de conglomérats de sébum artificiel et de poudre. A partir de ce moment, on ajoute le sébum artificiel à raison d'une goutte à la fois et on triture ensuite le mélange avec la spatule. On cesse l'addition de sébum artificiel lorsque l'on obtient une pâte ferme et lisse. Cette pâte doit se laisser étendre sur la plaque de verre sans craquelures ni formation de grumeaux. On note alors le volume Vs (exprimé en ml) de sébum artificiel utilisé.

La prise de sébum correspond au rapport Vs / m.

### Exemple 1 : Fond de teint compact

- Oxydes de fer jaune, brun, noir 5,75 g
- Talc 56,25 g
- Microsphères de silice amorphe : Sunsphère H-33 de Asahi Glass 10,00 g
- Microbilles de cire de carnauba : Microcare 350® de Micro Powders 20,00 g
- Polydécène hydrogéné : Ceraflow E® de Shamrock 3,00 g
- Polyméthyl-trifluoro-propyl diméthylsiloxane :
- X22-819® de Shinetsu 5,00 g

La composition est préparée en mélangeant l'ensemble des poudres (oxydes de fer, silice, cire de carnauba) puis en y ajoutant les huiles (polydécène hydrogéné, silicone fluorée), ce mélange est ensuite broyé et tamisé jusqu'à l'obtention d'un mélange homogène. Une partie de la composition est placée dans une coupelle puis compactée.

On obtient une poudre compacte dont l'application sur le visage procure un maquillage présentant une bonne matité au cours de la journée.

### Exemple 2 : Fond de teint

- Oxydes de fer jaune, brun, noir 3,23 g
- Microsphères de silice amorphe :
   Sunsphère® H-33 de Asahi - Glass 1,00 g
- Conservateurs qs
- Dioxyde de titane 10,55 g
- Sericite 15,00 g
- Nitrure de bore 6,00 g
- Talc 28,30 g
- Poudre de polyuréthane et de silice (98/2) :
   Plastic Powder® D-400 de Toshiki 3,00 g
- Dioxyde de titane - oxyde de zinc - talc enrobé
   méthylhydrogéno polysiloxane : TZ Powder® type 2 de Miyoshi 4,00 g
- Poudre de polydimethylsiloxane réticulé :
   Trefil Powder® E-506 C de Dow Corning 2,00 g
- Poudre de copolymère éthylène/acide acrylique :
   Flobeads® EA-209 de Sumitomo 5,00 g
- Microbilles de cire de paraffine :
   Microease® 114S de Micro Powders 10,00 g
   - Stéarate de magnésium 3,00 g
   - Polyméthyl-trifluoro-propyl diméthylsiloxane :
      X22-819® de Shin Etsu 2,92 g
   - P-méthoxy-4-cinnamate d'éthyl-2-hexyle 4,00 g
   - Isononanoate d'isononyle 1,20 g

### Exemple 3 : Fond de teint

- Oxydes de fer jaune, brun, noir 3,23 g
- Dioxyde de titane enrobé de lauroyl lysine (95/5) 9,00 g
- Dioxyde de titane ― oxyde de zinc ― talc enrobé
   méthylhydrogéno polysiloxane : TZ Powder® type 2 de Miyoshi 4,00 g
- Conservateurs qs
- Talc 30,80 g
- Microsphères de silice amorphe :
   Sunsphère H-33 de Asahi ― Glass 1,00 g
- Microbilles de silice :
   SILICA BEADS® SB150 de Miyoshi 1,00 g
- Poudre de polyuréthane et de silice (98/2) :
   Plastic Powder® D-400 de Toshiki 5,00 g
- Poudre de copolymère éthylène/acide acrylique :
   Flobeads® EA-209 de Sumitomo 5,00 g
- Poudre de nylon :
   ORGASOL® 2002 EXTRA D NAT COS 5,00 g
- Poudre de polydimethylsiloane réticulé :
   Trefil Powder® E-506 C de Dow Corning 2,00 g
- Microbilles de cire de paraffine :
   Microease® 114S de Micro Powders 8,00 g
- Séricite enrobé de cire de Carnauba (94,5/5,5) :
   Séricite UW-A5® de Toshiki 15,00 g
- Stéarate de magnésium 3,00 g
- Isononanoate d'Isononyle 1,20 g
- Polyméthyl-trifluoro-propyl diméthylsiloxane :
   X22-819® de Shin Etsu 2,00 g
- P-méthoxy-4-cinnamate d'éthyl-2-hexyle 4,00 g

## Revendications

1. Composition cosmétique sous forme de poudre compacte pour le maquillage et/ou le soin de la peau comprenant au moins un composé absorbant et/ou adsorbant le sébum et au moins une phase grasse, ladite phase grasse comprenant à raison de plus de 40 % en poids de son poids total une phase grasse solide, et ladite composition étant **caractérisée en ce que** la phase grasse totale est présente en une teneur inférieure ou égale à 35 % en poids par rapport au poids total de la composition, à l'exclusion d'une composition comprenant 10 % en poids de cire de Carnauba, 6 % en poids de stéarate de magnésium, et 5 % en poids de silicate de magnésium et d'aluminium amorphe par rapport au poids total de la composition.

2. Composition selon la revendication 1 **caractérisée en ce que** la phase grasse solide présente un point de fusion supérieur à 30°C, notamment allant de 30 à 250 °C et en particulier allant de 30 à 230°C.

3. Composition selon la revendication 1 ou 2 **caractérisée en ce que** la phase grasse solide est présente en une teneur supérieure ou égale à 45 % en poids, notamment supérieure ou égale à 50 % en poids, en particulier supérieure ou égale à 55 % en poids, voire supérieur ou égal à 60 % en poids, par rapport au poids total de la phase grasse.

4. Composition selon l'une quelconque des revendications 1 à 3 **caractérisée en ce que** la phase grasse solide comprend au moins un composé choisi parmi les cires, les savons métalliques et leurs mélanges.

5. Composition selon l'une quelconque des revendications 1 à 4 **caractérisée en ce que** la phase grasse solide comprend au moins une cire choisie parmi :
- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et notamment à plus de 55°C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges.

6. Composition selon l'une quelconque des revendications 1 à 5 **caractérisée en ce que** la phase grasse solide comprend au moins une cire choisie parmi les cires de paraffine, les cires de carnauba et leurs mélanges.

7. Composition selon l'une quelconque des revendications 1 à 6 **caractérisée en ce que** la phase grasse solide comprend au moins une cire présente dans ladite composition au moins partiellement sous forme de poudre, telle que les microbilles de cire de paraffine et les microbilles de cire de carnauba.

8. Composition selon l'une quelconque des revendications 1 à 7 **caractérisée en ce que** la phase grasse solide comprend au moins un composé choisi parmi le laurate de zinc, le stéarate de magnésium, le myristate de magnésium, le stéarate de zinc et leurs mélanges.

9. Composition selon l'une quelconque des revendications 1 à 8 **caractérisée en ce que** le rapport pondéral phase grasse solide / pompe à sébum est au moins égal à 1, et peut notamment varier de 1 à 2,5.

10. Composition selon l'une quelconque des revendication 1 à 9 **caractérisée en ce que** la phase grasse solide est présente en une teneur variant de 8 à 25 % en poids, notamment de 10 à 22 % en poids, et en particulier de 11 à 20 % en poids par rapport au poids total de la composition.

11. Composition selon l'une quelconque des revendication 1 à 10 **caractérisée en ce qu'**elle comprend au moins une phase grasse liquide.

12. Composition selon la revendication 11 **caractérisée en ce que** la phase grasse liquide comprend au moins une huile choisi parmi :
- l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures ;
- les esters gras ;
- les huiles de silicone ;
- les acides gras supérieurs ;
- les alcools gras supérieurs ;
- les poly méthylfluoroalkyl diméthylsiloxanes de formule (I) : 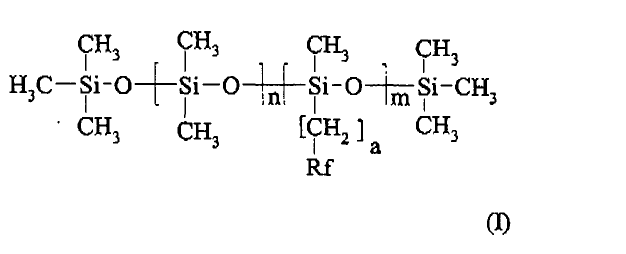
dans laquelle :
- n représente un entier variant de 5 à 90, notamment de 30 à 80 et en particulier de 50 à 80,
- m représente un entier variant de 1 à 150, notamment de 1 à 80, et en particulier de 1 à 40,
- a représente un entier variant de 0 à 5, et
- Rf désigne un radical perfluoroalkyle comprenant de 1 à 8 atomes de carbone, et
- leurs mélanges.

13. Composition selon la revendication 11 ou 12 **caractérisée en ce que** le rapport pondéral phase grasse liquide / pompe(s) à sébum est inférieur ou égal à 2 et peut notamment varier de 0,5 à 2.

14. Composition selon l'une quelconque des revendication 11 à 13 **caractérisée en ce que** la phase grasse liquide est présente en une teneur variant de 4 à 15 % en poids, notamment de 6 à 13 % en poids, et en particulier de 7 à 13 % en poids par rapport au poids total de la composition.

15. Composition selon l'une quelconque des revendication 1 à 14 **caractérisée en ce que** la phase grasse totale est présente en une teneur supérieure à 12 % en poids, et notamment variant de 15 à 30 % en poids par rapport au poids total de la composition.

16. Composition selon l'une quelconque des revendications 1 à 15, **caractérisée en ce que** le composé absorbant et/ou adsorbant le sébum permet une prise de sébum supérieure ou égale à 1 ml/g, notamment variant de 1 à 20 ml/g, et en particulier de 1 à 15 ml/g.

17. Composition selon l'une quelconque des revendications 1 à 16, **caractérisée en ce que** les particules de composé absorbant et/ou adsorbant le sébum présentent une surface spécifique BET supérieure ou égale à 300 m²/g, notamment supérieure ou égale à 500 m²/g, en particulier supérieure ou égale à 600 m²/g et notamment inférieure ou égale à 1500 m²/g.

18. Composition selon l'une quelconque des revendications 1 à 17, **caractérisée en ce que** les particules de composé absorbant et/ou adsorbant le sébum sont d'origine minérale ou organique.

19. Composition selon l'une quelconque des revendications 1 à 18, **caractérisée en ce que** les particules de composé absorbant et/ou adsorbant le sébum sont choisies parmi la silice, les poudres de polyamide, les poudres de polymères acryliques, notamment de polyméthacrylate de méthyle, de polyméthacrylate de méthyle/diméthacrylate d'éthylène glycol, de polyméthacrylate d'allyle/diméthacrylate d'éthylène glycol et de copolymère diméthacrylate d'éthylène glycol/méthacrylate de lauryle et les poudres de polyéthylène, notamment de polyéthylène/acide acrylique.

20. Composition selon l'une quelconque des revendications 1 à 19, **caractérisée en ce que** les particules du composé absorbant et/ou adsorbant le sébum sont traitées en surface par au moins un agent de traitement hydrophobe.

21. Composition selon l'une quelconque des revendications 1 à 20, **caractérisée en ce que** le composé absorbant et/ou adsorbant le sébum est présent en une teneur variant de 2 à 18 % en poids, notamment de 4 à 18 % en poids, et en particulier de 5 à 15 % en poids, par rapport au poids total de la composition.

22. Composition selon l'une quelconque des revendications 1 à 21, **caractérisée en ce qu'**elle comprend au moins une association pompes à sébum/phase grasse solide choisie parmi :
- microsphères de silice poreuses/cire de carnauba ou de paraffine micronisée,
- microsphères de silice poreuses et poudre de polyéthylène/acide acrylique/cire de carnauba ou de paraffine micronisée et stéarate de magnésium ou stéarate de zinc, et
- microsphères de silice poreuses, poudre de polyéthylène/acide acrylique et poudre de polyamide/cire de carnauba ou de paraffine micronisée, et stéarate de magnésium ou stéarate de zinc.

23. Composition selon l'une quelconque des revendications 1 à 22, **caractérisée en ce qu'**elle comprend en outre au moins une charge additionnelle.

24. Composition selon la revendication 23, **caractérisée en ce que** la charge additionnelle est choisie parmi le talc, le mica, la silice, le kaolin, les poudres de polyamide, de poly-β-alanine et de polyéthylène, les poudres de polymères de tétrafluoroéthylène, la lauroyl-lysine, l'amidon, le nitrure de bore, les poudres de polymères d'acide acrylique, les microbilles de résine de silicone, le carbonate de calcium précipité, le carbonate et l'hydrocarbonate de magnésium, l'hydroxyapatite, les microsphères de silice creuses et les microcapsules de céramique.

25. Composition selon la revendication 23 ou 24, **caractérisée en ce que** la charge est présente en une teneur variant de 40 à 95 % en poids, notamment de 45 à 85 % en poids, et en particulier de 45 à 70 % en poids, par rapport au poids total de la composition.

26. Composition selon l'une quelconque des revendications 1 à 25, **caractérisée en ce qu'**elle comprend en outre au moins une matière colorante pulvérulente.

27. Composition selon la revendication 26, **caractérisée en ce que** la matière colorante pulvérulente est choisie parmi les pigments et les nacres.

28. Composition selon la revendication 26 ou 27, **caractérisée en ce que** la matière colorante pulvérulente est présente en une teneur variant de 0,5 à 30 % en poids, notamment de 1 à 22 % en poids, et particulier de 3 à 18 % en poids, par rapport au poids total de la composition.

29. Composition selon l'une quelconque des revendications 1 à 28, **caractérisée en ce qu'**elle comprend en outre, des agents colorants liposolubles ou hydrosolubles, des conservateurs, des actifs cosmétiques, des agents hydratants, des filtres UV, des épaississants, de l'eau, des tensioactifs et/ou des parfums.

30. Composition selon l'une quelconque des revendications 1 à 29, **caractérisée en ce qu'**elle se présente sous la forme d'un fard à joues, fard à paupières, d'une poudre pour le visage, d'un fond de teint, d'un produit anti-cemes, d'un produit de maquillage du corps, d'un produit de soin pour le visage ou d'un produit de soin du corps.

31. Composition selon la revendication 30, **caractérisée en ce qu'**il s'agit d'un fond de teint.

32. Utilisation d'au moins une phase grasse solide et d'au moins un composé absorbant et/ou adsorbant le sébum dans une composition cosmétique de type poudre compacte pour obtenir un maquillage homogène en couleur et/ou en matité, ladite composition cosmétique comprenant au moins une phase grasse comprenant, à raison de plus de 40 % en poids de son poids total, une phase grasse solide, ladite composition étant également **caractérisée en ce que** la phase grasse totale est présente en une teneur inférieure ou égale à 35 % en poids par rapport au poids total de la composition.

33. Utilisation selon la revendication 32, **caractérisée en ce que** le rapport pondéral phase grasse solide / pompe(s) à sébum est au moins égal à 1, et peut notamment varier de 1 à 2,5.

34. Utilisation selon la revendication 32 ou 33, **caractérisée en ce que** la phase grasse solide est telle que définie selon l'une quelconque des revendications 4 à 10.

35. Utilisation selon l'une quelconque des revendication 32 à 34, **caractérisée en ce que** le composé absorbant et/ou adsorbant le sébum est tel que défini en revendications 16 à 20.

36. Procédé de maquillage et/ou de soin de la peau comprenant au moins une étape d'application sur la peau d'au moins une composition selon l'une quelconque des revendications 1 à 31.

37. Utilisation d'une composition selon l'une quelconque des revendications 1 à 31 pour obtenir un maquillage homogène en couleur et/ou en matité.

## Claims

1. Cosmetic composition in the form of a compact powder for making up and/or caring for the skin, comprising at least one compound absorbing and/or adsorbing sebum and at least one fatty phase, the said fatty phase comprising a solid fatty phase in an amount of more than 40% by weight of its total weight and said composition being **characterized in that** the total fatty phase is present in an amount of less or equal to 35% by weight, relative to the total weight of the composition excluding a composition comprising 10% by weight of Carnauba wax, 6% by weight of magnesium stearate, and 5% by weight of amorphous aluminium and magnesium silicate relative to the total weight of the composition.

2. Composition according to Claim 1, **characterized in that** the solid fatty phase has a melting point greater than 30°C, especially ranging from 30 to 250°C and in particular ranging from 30 to 230°C.

3. Composition according to Claim 1 or 2, **characterized in that** the solid fatty phase is present in an amount greater than or equal to 45% by weight, especially greater than or equal to 50% by weight, in particular greater than or equal to 55% by weight, or even greater than or equal to 60% by weight, relative to the total weight of the fatty phase.

4. Composition according to any one of Claims 1 to 3, **characterized in that** the solid fatty phase comprises at least one compound chosen from waxes, metallic soaps and mixtures thereof.

5. Composition according to any one of Claims 1 to 4, **characterized in that** the solid fatty phase comprises at least one wax chosen from:
- beeswax, lanolin wax, Chinese waxes; rice wax, Carnauba wax, Candelilla wax, Ouricury wax, cork fibre wax, sugar cane wax, Japan wax and sumac wax; Montana wax, microcrystalline waxes, paraffin waxes, ozokerites, ceresin wax, lignite wax, polyethylene waxes, the waxes obtained by Fisher-Tropsch synthesis, fatty acid esters and glycerides which are concrete at 40°C and in particular at more than 55°C,
- the waxes obtained by catalytic hydrogenation of animal or vegetable oils having linear or branched **C₈-C₃₂** fatty chains, in particular hydrogenated jojoba oil, hydrogenated sunflower oil, hydrogenated castor oil, hydrogenated copra oil and hydrogenated lanolin oil,
- silicone waxes or fluorinated waxes, and
- mixtures thereof.

6. Composition according to any one of Claims 1 to 5, **characterized in that** the solid fatty phase comprises at least one wax chosen from paraffin waxes, Carnauba waxes and mixtures thereof.

7. Composition according to any one of Claims 1 to 6, **characterized in that** the solid fatty phase comprises at least one wax which is present in the said composition at least partially in powdered form, such as microbeads of paraffin wax and microbeads of Carnauba, wax.

8. Composition according to any one of Claims 1 to 7, **characterized in that** the solid fatty phase comprises at least one compound chosen from zinc laurate, magnesium stearate, magnesium myristate, zinc stearate and mixtures thereof.

9. Composition according to any one of Claims 1 to 8, **characterized in that** the solid fatty phase/sebum pump weight ratio is at least equal to 1, and may vary in particular from 1 to 2.5.

10. Composition according to any one of Claims 1 to 9, **characterized in that** the solid fatty phase is present in an amount varying from 8 to 25% by weight, especially from 10 to 22% by weight, and in particular from 11 to 20% by weight relative to the total weight of the composition.

11. Composition according to any one of Claims 1 to 10, **characterized in that** it comprises at least one liquid fatty phase.

12. Composition according to Claim 11, **characterized in that** the liquid fatty phase comprises at least one oil chosen from:
- mink oil, turtle oil, soya bean oil, grapeseed oil, sesame oil, maize oil, rapeseed oil, sunflower oil, cottonseed oil, avocado oil, olive oil, castor oil, jojoba oil, groundnut oil;
- hydrocarbon oils;
- fatty esters;
- silicone oils;
- higher fatty acids;
- higher fatty alcohols;
- polymethylfluoroalkyl dimethylsiloxanes of formula (1): 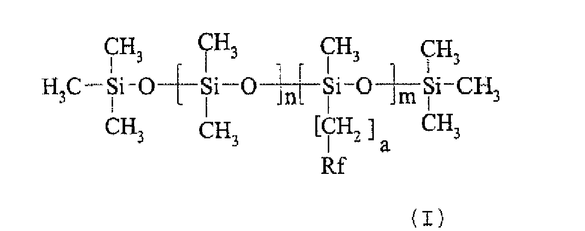 in which:
- n represents an integer varying from 5 to 90, especially from 30 to 80 and in particular from 50 to 80,
- m represents an integer varying from 1 to 150, especially from 1 to 80, and in particular from 1 to 40,
- a represents an integer varying from 0 to 5, and
- Rf denotes a perfluoroalkyl radical comprising from 1 to 8 carbon atoms; and
- mixtures thereof.

13. Composition according to Claim 11 or 12, **characterized in that** liquid fatty phase/sebum pump(s) weight ratio is less than or equal to 2 and may vary in particular from 0.5 to 2.

14. Composition according to any one of Claims 11 to 13, **characterized in that** the liquid fatty phase is present in an amount varying from 4 to 15% by weight, in particular from 6 to 13% by weight, and in particular from 7 to 13% by weight relative to the total weight of the composition.

15. Composition according to any ane of Claims 1 to 14, **characterized in that** the total fatty phase is present in an amount superior to 12 by weight, and especially varying from 15 to 30% by weight relative to the total weight of the composition.

16. Composition according to any one of Claims 1 to 15, **characterized in that** the compound absorbing and/or adsorbing sebum allows a sebum uptake greater than or equal to 1 ml/g, especially varying from 1 to 20 ml/g, and in particular from 1. to 15 ml/g.

17. Composition according to any one of Claims 1 to 16, **characterized in that** the particles of compound absorbing and/or adsorbing sebum have a BET specific surface area greater than or equal to 300 m²/g, especially greater than or equal to 500 m²/g, in particular greater than or equal to 600 m²/g and especially less than or equal to 1500 m²/g.

18. Composition according to any one of Claims 1 to 17, **characterized in that** the particles of compound absorbing and/or adsorbing sebum are of inorganic organic origin.

19. Composition according to any one of Claims 1 to 18, **characterized in that** the particles of compound absorbing and/or adsorbing sebum are chosen from silica, polyamide powders, powders of acrylic polymers, in particular of polymethyl methacrylate, polymethyl methacrylate/ethylene glycol dimethacrylate, of polyallyl methacrylate/ethylene glycol dimethacrylate and of ethylene glycol dimethacrylate/lauryl methacrylate copolymer and powders of polyethylene, in particular of polyethylene/acrylic acid.

20. Composition according to any one of Claims 1 to 19, **characterized in that** the particles of compound absorbing and/or adsorbing sebum are surface-treated with at least one hydrophobic treatment agent.

21. Composition according to any one of Claims 1 to 20, **characterized in that** the compound absorbing and/or adsorbing sebum is present in an amount varying from 2 to 18% by weight, especially from 4 to 18% by weight, and in particular from 5 to 15% by weight, relative to the total weight of the composition.

22. Composition according to any one of Claims 1 to 21, **characterized in that** it comprises at least one sebum pump/solid fatty phase combination chosen from:
- porous silica microspheres/Carnauba or micronized paraffin wax,
- porous silica microspheres and acrylic acid/polyethylene powder/Carnauba or micronized paraffin wax and magnesium stearate or zinc stearate, and
- porous silica microspheres, polyethylene/ acrylic acid powder and polyamide powder/ Carnauba or micronized paraffin wax, and magnesium stearate or zinc stearate.

23. Composition according to any one of Claims 1 to 22, **characterized in that** it additionally comprises at least one additional filler.

24. Composition according to Claim 23, **characterized in that** the additional filler is chosen from talc, mica, silica, kaolin, powders of polyamide, of poly-β-alanine and of polyethylene, powders of polymers of tetrafluoroethylene, lauryllysine, starch, boron nitride, powders of acrylic acid polymers, microbeads of silicone resin, precipitated calcium carbonate, magnesium carbonate and hydrocarbonate, hydroxyapatite, hollow silica microspheres, and ceramic microcapsules.

25. Composition according to Claim 23 or 24, **characterized in that** the filler is present in an amount varying from 40 to 95% by weight, especially from 45 to 85% by weight, and in particular from 45 to 70% by weight, relative to the total weight of the composition.

26. Composition according to any one of Claims 1 to 25, **characterized in that** it additionally comprises at least one pulverulent colouring material.

27. Composition according to Claim 26, **characterized in that** the pulverulent colouring material is chosen from pigments and pearlescent agents.

28. Composition according to Claim 26 or 27, **characterized in that** the pulverulent colouring material is present in an amount varying from 0.5 to 30% by weight, especially from 1 to 22% by weight, and in particular from 3 to 18% by weight, relative to the total weight of the composition.

29. Composition according to any one of Claims 1 to 28, **characterized in that** it additionally comprises fat-soluble or water-soluble colouring agents, preservatives, cosmetic active agents, moisturizing agents, UV-screening agents, thickeners, water, surfactants and/or perfumes.

30. Composition according to any one of Claims 1 to 29, **characterized in that** it is present in the form of a blusher, an eyeshadow, a face powder, a foundation, a concealer product, a make-up product for the body, a care product for the face or a care product for the body.

31. Composition according to Claim 30, **characterized in that** it is a foundation.

32. Use of at least one solid fatty phase and of at least one compound absorbing and/or adsorbing sebum in a cosmetic composition of the compact powder type to obtain a make-up which is homogeneous in colour and/or in mattness, said cosmetic composition comprising at least one fatty phase comprising a solid fatty phase in an amount of more than 40% by weight of its total, a solid fatty phase, said composition being also **characterized in** the fact that total fatty phase is present in an amount of less or equal to 35% by weight relative to the total weight of the composition.

33. Use according to Claim 32, **characterized in that** the solid fatty phase/sebum pump(s) weight ratio is at least equal to 1, and may in particular vary from 1 to 2.5.

34. Use according to Claim 32 or 33, **characterized in that** the solid fatty phase is as defined according to any one of Claims 4 to 10.

35. Use according to any one of Claims 32 to 34, **characterized in that** the compound absorbing and/or adsorbing sebum is as defined in Claims 16 to 20.

36. Method for making up and/or caring for the skin, comprising at least one step of applying to the skin at least one composition according to any one of Claims 1 to 31.

37. Use of a composition according to any one of Claims 1 to 31, to obtain a make-up which is homogeneous in colour and/or in mattness.

## Patentansprüche

1. Kosmetische Zusammensetzung in Form von Kompaktpuder zum Schminken und/oder zur Pflege der Haut, die mindestens eine das Sebum absorbierende und/oder adsorbierende Verbindung und mindestens eine Fettphase umfasst, wobei die Fettphase mehr als 40 Gew.-% ihres Gesamtgewichts entsprechend von einer festen Fettphase umfasst, wobei die Zusammensetzung **dadurch gekennzeichnet ist, dass** die gesamte Fettphase in einem Gehalt von kleiner oder gleich 35 Gew.-%, bezogen auf das Gesamtgewicht der Zusammensetzung, vorhanden ist, mit Ausschluss einer Zusammensetzung, die 10 Gew.% Carnaubawachs, 6 Gew.-% Magnesiumstearat und 5 Gew.% amorphes Magnesium- und Aluminiumsilikat, bezogen auf das Gesamtgewicht der Zusammensetzung, umfasst.

2. Zusammensetzung nach Anspruch 1, **dadurch gekennzeichnet, dass** die feste Fettphase einen Schmelzpunkt von größer 30 °C, insbesondere im Bereich von 20 bis 250 °C und speziell von 30 bis 230 °C aufweist.

3. Zusammensetzung nach Anspruch 1 oder 2, **dadurch gekennzeichnet, dass** die feste Fettphase in einem Gehalt von größer oder gleich 45 Gew.-%, insbesondere von größer oder gleich 50 Gew.-%, speziell von größer oder gleich 55 Gew.-%, sogar von größer oder gleich 60 Gew.-%, bezogen auf das das Gesamtgewicht der Fettphase, vorhanden ist.

4. Zusammensetzung nach einem der Ansprüche 1 bis 3, **dadurch gekennzeichnet, dass** die feste Fettphase mindestens eine Verbindung umfasst, die aus Wachsen, Metallseifen und ihren Gemischen ausgewählt ist.

5. Zusammensetzung nach einem der Ansprüche 1 bis 4, **dadurch gekennzeichnet, dass** die feste Fettphase mindestens ein Wachs umfasst, das ausgewählt ist aus:
- Bienenwachs, Lanolinwachs, chinesischen Insektenwachsen; Reiskleiewachs, Carnaubawachs, Candellila-Wachs, Ouricurywachs, Wachs von Korkfasern, Wachs von Zuckerrohr, Japan-Wachs und Sumach-Wachs; Montanwachs, mikrokristallinen Wachsen, Paraffinwachsen, Ozokeriten, Kerosinwachs, Lignitwachs, Polyethylenwachsen, Wachsen, die durch Fischer-Tropsch-Synthese erhalten werden, Estern von Fettsäuren und den bei 40 °C und insbesondere bei über 55 °C festen Glyceriden,
- Wachsen, die erhalten werden durch katalytische Hydrierung von tierischen oder pflanzlichen Ölen mit linearen oder verzweigten C₈-C₃₂-Fettsäureketten, insbesondere hydriertes Jojobaöl, hydriertes Sonnenblumenöl, hydriertes Rizinusöl, hydriertes Coprah-Öl und hydriertes Lanolinöl,
- silikonierten oder fluorierten Wachsen, und
- ihren Gemischen.

6. Zusammensetzung nach einem der Ansprüche 1 bis 5, **dadurch gekennzeichnet, dass** die feste Fettphase mindestens ein Wachs umfasst, das aus Paraffinwachsen, Carnaubawachsen und ihren Gemischen ausgewählt ist.

7. Zusammensetzung nach einem der Ansprüche 1 bis 6, **dadurch gekennzeichnet, dass** die feste Fettphase mindestens ein Wachs umfasst, das in der Zusammensetzung mindestens zum Teil in der Form von Pulver vorhanden ist, wie Paraffinwachs-Mikrobeads und Carnaubawachs-Mikrobeads.

8. Zusammensetzung nach einem der Ansprüche 1 bis 7, **dadurch gekennzeichnet, dass** die feste Fettphase mindestens eine Verbindung umfasst, die aus Zinklaurat, Magnesiumstearat, Magnesiummyristat, Zinkstearat und ihren Gemischen ausgewählt ist.

9. Zusammensetzung nach einem der Ansprüche 1 bis 8, **dadurch gekennzeichnet, dass** das Gewichtsverhältnis feste Fettphase/Sebumpumpe mindestens gleich 1 ist und insbesondere von 1 bis 2,5 variieren kann.

10. Zusammensetzung nach einem der Ansprüche 1 bis 9, **dadurch gekennzeichnet, dass** die feste Fettphase in einem Gehalt vorhanden ist, der von 8 bis 25 Gew.-%, insbesondere von 10 bis 22 Gew.-% und speziell von 11 bis 20 Gew.-% variiert, bezogen auf das Gesamtgewicht der Zusammensetzung.

11. Zusammensetzung nach einem der Ansprüche 1 bis 10, **dadurch gekennzeichnet, dass** sie mindestens eine flüssige Fettphase umfasst.

12. Zusammensetzung nach Anspruch 11, **dadurch gekennzeichnet, dass** die flüssige Fettphase mindestens ein Öl umfasst, das ausgewählt ist aus:
- Nerzöl, Schildkrötenöl, Sojaöl, Trauhenkernöl, Sesamöl, Maisöl, Colzaöl, Sonnenblumenöl, Baumwollöl, Avocadoöl, Olivenöl, Rizinusöl, Jojobaöl, Erdnussöl;
- Ölen von Kohlenwasserstoffen;
- Fettsäureestern;
- Siliconölen;
- höhere Fettsäure;
- höheren Fettalkoholen;
- Polymethylfluoralkyldimethylsiloxanen der Formel (I): 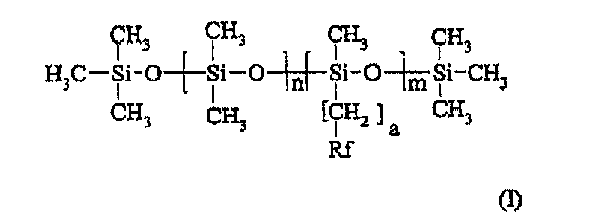 wobei
- n eine ganze Zahl darstellt, die von 5 bis 90, insbesondere von 30 bis 80 und speziell von 50 bis 80 variiert,
- m eine ganze Zahl darstellt, die von 1 bis 150, insbesondere von 1 bis 80 und speziell von 1 bis 40 variiert,
- a eine ganze Zahl darstellt, die von 0 bis 5 variiert, und
- Rf einen Perfluoralkylrest bezeichnet, der 1 bis 8 Kohlenstoffatome einschließt, und
- ihren Gemischen.

13. Zusammensetzung nach Anspruche 11 oder 12, **dadurch gekennzeichnet, dass** das Gewichtsverhältnis flüssige Fettphase/Sebum-Pumpe(n) kleiner oder gleich 2 ist und insbesondere von 0,5 bis 2 variieren kann.

14. Zusammensetzung nach einem der Ansprüche 11 bis 13, **dadurch gekennzeichnet, dass** die flüssige Fettphase in einem Gehalt vorhanden ist, der von 4 bis 15 Gew.-%, insbesondere von 6 bis 13 Gew.-%, speziell von 7 bis 13 Gew.-%, bezogen auf das Gesamtgewicht der Zusammensetzung, variiert.

15. Zusammensetzung nach einem der Ansprüche 1 bis 14, **dadurch gekennzeichnet, dass** die gesamte Fettphase in einem Gehalt von über 12 Gew.% und insbesondere variierend von 15 bis 30 Gew.-% vorhanden ist, bezogen auf das Gesamtgewicht der Zusammensetzung.

16. Zusammensetzung nach einem der Ansprüche 1 bis 15, **dadurch gekennzeichnet, dass** die das Sebum absorbierende und/oder adsorbierende Verbindung eine Sebum-Aufnahme von größer oder gleich 1 ml/g, insbesondere variierend von 1 bis 20 ml/g und speziell von 1 bis 15 ml/g erlaubt.

17. Zusammensetzung nach einem der Ansprüche 1 bis 16, **dadurch gekennzeichnet, dass** die Teilchen von das Sebum absorbierender und/oder adsorbierender Verbindung eine spezifische BET-Oberfläche von größer oder gleich 300 m²/g, insbesondere von größer oder gleich 500 m²/g, speziell von größer oder gleich 600 m²/g und insbesondere von kleiner oder gleich 1500 m²/g aufweisen.

18. Zusammensetzung nach einem der Ansprüche 1 bis 17, **dadurch gekennzeichnet, dass** die Teilchen von das Sebum absorbierender und/oder adsorbierender Verbindung mineralischen oder organischen Ursprungs sind.

19. Zusammensetzung nach einem der Ansprüche 1 bis 18, **dadurch gekennzeichnet, dass** die Teilchen von das Sebum absorbierender und/oder adsorbierender Verbindung ausgewählt sind aus Kieselsäure, Polyamidpulvern, Pulvern von Acrylpolymeren, insbesondere von Polymethylmethacrylat, Polymethylmethacrylat/Ethylenglycoldimethacrylat, Polyallylmethacrylat/Ethylenglycoldimethacrylat und von Ethylenglycoldimethacrylat/Laurylmethacrylat-Coploymer, und Polyethylenpulvem, insbesondere von Polyethylen/Acrylsäure.

20. Zusammensetzung nach einem der Ansprüche 1 bis 19, **dadurch gekennzeichnet, dass** die Teilchen von das Sebum absorbierender und/oder adsorbierender Verbindung durch mindestens ein hydrophobes Behandlungsmittel oberflächenbehandelt wurden.

21. Zusammensetzung nach einem der Ansprüche 1 bis 20, **dadurch gekennzeichnet, dass** die das Sebum absorbierende und/oder adsorbierende Verbindung in einem Gehalt vorhanden ist, der von 2 bis 18 Gew.%, insbesondere von 4 bis 18 Gew.-% und speziell von 5 bis 15 Gew.% variiert, bezogen auf das Gesamtgewicht der Zusammensetzung.

22. Zusammensetzung nach einem der Ansprüche 1 bis 21, **dadurch gekennzeichnet, dass** sie mindestens eine Kombination Sebumpumpen/feste Fettphase umfasst, die ausgewählt ist aus:
- porösen Siliciumdioxid-MikrobeadslCarnaubawachs oder mikronisiertem Paraffin,
- porösen Siliciumdioxid-Mikrobeads und Pulver von Polyethylen/Acrylsäure/ Carnaubawachs oder von mikronisiertem Paraffin und Magnesiumstearat oder Zinkstearat, und
- porösen Siliciumdioxid-Mikrobeads, Pulver von Polyethylen/Acrylsäure und Pulver von Polyamid/Carnaubawachs oder von mikronisiertem Paraffin, und Magnesiumstearat oder Zinkstearat.

23. Zusammensetzung nach einem der Ansprüche 1 bis 22, **dadurch gekennzeichnet, dass** sie weiterhin mindestens einen zusätzlichen Füllstoff einschließt.

24. Zusammensetzung nach Anspruch 23, **dadurch gekennzeichnet, dass** der zusätzliche Füllstoff ausgewählt ist aus Talk, Mica, Kieselsäure, Kaolin, Pulvern von Polyamid, von Poly-β-alanin und von Polyethylen, Pulvern von Tetrafluorethylen-Polymeren, Lauroyllysin, Amidon, Bornitrid, Pulvern von Acrylsäure-Polymeren, Siliconharz-Mikrobeads, präzipitiertem Calciumcarbonat Magnesiumcarbonat und -hydrogencarbonat, Hydroxylapatit, hohlen Siliciumdioxid-Mikrobeads und keramischen Mikrokapseln.

25. Zusammensetzung nach Anspruch 23 oder 24, **dadurch gekennzeichnet, dass** der Füllstoff in einem Gehalt vorhanden ist, der von 40 bis 95 Gew.%, insbesondere von 45 bis 85 Gew.% und speziell von 45 bis 70 Gew.-% variiert, bezogen auf das Gesamtgewicht der Zusammensetzung.

26. Zusammensetzung nach einem der Ansprüche 1 bis 25, **dadurch gekennzeichnet, dass** sie weiterhin mindestens einen pulverisierten Farbstoff umfasst.

27. Zusammensetzung nach Anspruch 26, **dadurch gekennzeichnet, dass** der pulverisierte Farbstoff aus Pigmenten und Perlmuttlacken ausgewählt ist,

28. Zusammensetzung nach Anspruch 26 oder 27, **dadurch gekennzeichnet, dass** der pulverisierte Farbstoff in einem Gehalt vorhanden ist, der von 0,5 bis 30 Gew,-%, insbesondere von 1 bis 22 Gew.-%, speziell von 3 bis 18 Gew,% variiert, bezogen auf das Gesamtgewicht der Zusammensetzung.

29. Zusammensetzung nach einem der Ansprüche 1 bis 28, **dadurch gekennzeichnet, dass** sie weiterhin fett- oder wasserlösliche Farbstoffe, Konservierungsmittel, kosmetische Wirkstoffe, hydratisierende Mittel, UV-Filter, Verdicker, Wasser, Tenside und/oder Duftstoffe umfasst.

30. Zusammensetzung nach einem der Ansprüche 1 bis 29, **dadurch gekennzeichnet, dass** sie in Form eines Wangenrouge, eines Lidschattens, eines Gesichtspuders, eines Fond de Teint, eines Antifaltenprodukts, eines Körperschminkprodukts, eines Pflegeprodukts für das Gesicht oder eines Körperpflegeprodukts vorliegt.

31. Zusammensetzung nach Anspruch 30, **dadurch gekennzeichnet, dass** es sich um einen Fond de Teint handelt.

32. Verwendung von mindestens einer festen Fettphase und von mindestens einer das Sebum absorbierenden und/oder adsorbierenden Verbindung in einer kosmetischen Zusammensetzung vom Typ eines Kompaktpuders zum Erhalt einer homogenen Farb- oder Mattierungsschminke, wobei die kosmetische Zusammensetzung mindestens eine Fettphase umfasst, die mehr als 40 Gew.-% ihres Gesamtgewichts entsprechend von einer festen Fettphase umfasst, wobei die Zusammensetzung auch **dadurch gekennzeichnet ist, dass** die gesamte Fettphase in einem Gehalt von kleiner oder gleich 35 Gew.-%, bezogen auf das Gesamtgewicht der Zusammensetzung, vorhanden ist.

33. Verwendung nach Anspruch 32, **dadurch gekennzeichnet, dass** das Gewichtsverhältnis feste Fettphase/Sebumpumpe(n) mindestens gleich 1 beträgt und insbesondere von 1 bis 2,5 variieren kann.

34. Verwendung nach Anspruch 32 oder 33, **dadurch gekennzeichnet, dass** die feste Fettphase so ist, wie nach einem der Ansprüche 4 bis 10 definiert.

35. Verwendung nach einem der Ansprüche 32 bis 34, **dadurch gekennzeichnet, dass** die das Sebum absorbierende und/oder adsorbierende Verbindung so ist, wie nach einem der Ansprüche 16 bis 20 definiert.

36. Verfahren zum Schminken und/oder zur Pflege der Haut, umfassend mindestens einen Schritt des Aufbringens auf die Haut von mindestens einer Zusammensetzung nach einem der Ansprüche 1 bis 31.

37. Verwendung einer Zusammensetzung nach einem der Ansprüche 1 bis 31 zum Erhalt einer homogenen Farb- oder Mattierungsschminke.
